(19) Europäisches Patentamt
European Patent Office
Office européen des brevets

(11) Veröffentlichungsnummer: **0 121 847**
**B1**

(12) # EUROPÄISCHE PATENTSCHRIFT

(45) Veröffentlichungstag der Patentschrift: **14.01.87**

(21) Anmeldenummer: **84103291.5**

(22) Anmeldetag: **26.03.84**

(51) Int. Cl.⁴: **C 07 C 121/75**, C 07 C 69/743, C 07 C 61/40, C 07 C 49/227, C 07 C 45/72, A 01 N 53/00

(54) **2,2-Dimethyl-3-(2-halogen-vinyl)-cyclopropancarbonsäure-ester, Verfahren zu ihrer Herstellung und ihrer Verwendung als Schädlingsbekämpfungsmittel.**

(30) Priorität: **07.04.83 DE 3312543**

(43) Veröffentlichungstag der Anmeldung: **17.10.84 Patentblatt 84/42**

(45) Bekanntmachung des Hinweises auf die Patenterteilung: **14.01.87 Patentblatt 87/3**

(84) Benannte Vertragsstaaten: **AT BE CH DE FR GB IT LI NL**

(56) Entgegenhaltungen:
**EP-A-0 009 709**
**EP-A-0 095 047**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Becker, Benedikt, Dr., Metzkausenerstrasse 14, D-4020 Mettmann (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

**EP 0 121 847 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue 2,2-Dimethyl-3-(2-halogen-vinyl)-cyclopropancarbonsäureester, ein Verfahren zu ihrer Herstellung und ihre Verwendung als Schädlingsbekämpfungsmittel, insbesondere als Insektizide und Akarizide.

Es ist bereits bekannt, daß 2,2-Dimethyl-3-vinylcyclo-propancarbonsäureester, wie z.B. 3-(2,2-Dichlorvinyl)-2,2-dimethyl-cyclopropancarbonsäure-3-phenoxy-benzyl-ester, 3-(2,2-Dichlor-vinyl)-2,2-dimethyl-cyclopropan-carbonsäure-3-phenoxy-α-cyano-benzylester, 2,2-Dimethyl-3-hex-1-en-cyclopropancarbonsäure-4-fluor-3-phenoxy-αcyano-benzylester und 3-[2-Chlor-2-(4-chlorphenyl)]-2,2-dimethyl-cyclopropancarbonsäure-4-fluor-3-phenoxy-αcyano-benzylester insektizid und akarizid wirksam sind (DE-OS 23 26 077, 31 10 725 und DE-OS 27 30 515)-.

Die Wirkung dieser Verbindungen ist jedoch bei niedrigen Aufwandkonzentrationen nicht immer voll befriedigend.

Weiterhin sind aus der EP-A 0 009 709 ähnlich strukturierte Cyclopropancarbonsäureester bekannt, die jedoch den erfindungsgemäßen Wirkstoffen biologisch unterlegen sind.

Es wurden nun neue 2,2-Dimethyl-3-(2-halogen-vinyl)-cyclopropancarbonsäureester der Formel (I) gefunden,

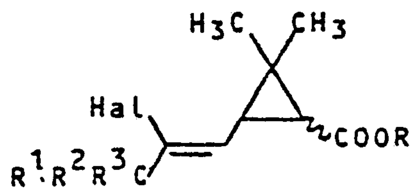

(I)

in welcher

$R^1$ und $R^2$ gleich oder verschieden sind und für Alkyl oder Halogenalkyl stehen,

$R^3$ für Alkyl oder für die Gruppierung $-(CH_2)_nR^4$ steht, in welcher

n für 0 oder 1 steht,

$R^4$ für Halogenalkyl, für gegebenenfalls durch Fluor, Chlor, Brom, Methyl, tert.-Butyl, Methoxy, Methylthio, Trifluorrnethyl und/oder Trifluormethoxy substituiertes Phenyl oder für die Gruppierung $-XR^5$ steht,

in welcher

X für Sauerstoff oder Schwefel steht und $R^5$ für Alkyl, Halogenalkyl, für gegebenenfalls durch Fluor, Chlor, Brom, Methyl, tert.-Butyl, Methoxy, Methylthio, Trifluorrnethyl und/oder Trifluorrnethoxy substituiertes Phenyl oder Phenylalkyl steht,

Hal für Fluor, Chlor oder Brom steht und

R für einen Rest $-CHR^6R^7$ steht, in welchem $R^6$ für wasserstoff oder CN steht und $R^7$ für 3-Phenoxy-phenyl, 4-Fluor-4-Phenoxyphenyl oder für Penta- fluorphenyl steht.

Die allgemeine Formel (I) schließt die möglichen Stereoisomeren und optische Isomeren sowie deren Mischungen mit ein.

Man erhält die neuen Verbindungen der Formel (I), wenn man 2,2-Dimethyl-3-(2-halogen-vinyl)-cyclopropancarbonsäuren der Formel (II),

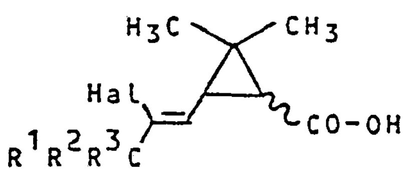

(II)

in welcher

$R^1$ $R^2$, $R^3$ und Hal die oben angegebenen Bedeutungen haben,

oder reaktionsfähige Derivate derselben

mit Alkoholen der Formel (III),

R-OH (III)

in welcher

R die oben angegebene Bedeutung hat,

oder mit reaktionsfähigen Derivaten derselben, gegebenenfalls in Gegenwart von Säureakzeptoren, gegebenenfalls in Gegenwärt von Katalysatoren und gegebenenfalls in Gegenwart von Verdünnungsmitteln umsetzt.

Die neuen 2,2-Dimethyl-3-(2-halogen-vinyl)-cyclopropan-carbonsäureester zeichnen sich durch hohe pestizide, insbesondere insektizide und akarizide Wirkung, und eine niedrige Fischtoxizität aus.

Ueberraschenderweise zeigen die erfindungsgemäßen Verbindungen der Formel (I) eine höhere insektizide und akarizide Wirkung als die bereits erwähnten 2,2-Dimethyl-3-vinyl-cyclopropancarbonsäureester, wie z.B. 3-(2,2-Dichlor-vinyl)-2,2-dimethyl-cyclopropancarbonsäure-3-phenoxy-benzylester, 3-(2,2 Dichlor-vinyl)-2,2-dimethyl-cyclo-propancarbonsäure-3-phenoxy-α-cyano-benzylester, 2,2-Dimethyl-3-hex-1-en-cyclopropancarbonsäure-4-fluor-3-phenoxy-α-cyano-benzylester und 3-[2-Chlor-2-(4-chlorphenyl)]-2,2-dimethyl-cyclopropancarbonsäure-4-fluor-3-phenoxy-α-cyano-benzylester.

Die Erfindung betrifft vorzugsheise Verbindungen der Formel (I), in welcher

$R^1$ und $R^2$ gleich oder verschieden sind und für Alkyl mit 1 bis 6 Kohlenstoffatomen und Halogenalkyl mit 1 bis 4 Kohlenstoffatomen im Alkylteil und 1 bis 5 Fluor, Chlor und/oder Bromatomen stehen,

$R^3$ für Alkyl mit 1 bis 12 Kohlenstoffatomen oder für die Gruppierung $-(CH_2)_nR^4$ steht,

in welcher

n für 0 oder 1 steht und

$R^4$ für Halogenalkyl mit I bis 4 Kohlenstoffatomen im Alkylteil und I bis 5 gleichen oder verschiedenen Halogenatomen,für gegebenenfalls durch Fluor, Chlor, Brom, Methyl, tert.-Butyl, Methoxy, Methylthio, Trifluormethyl und/oder Trifluormethoxy substituiertes Phenyl oder für die Gruppierung $XR^5$ steht,

in welcher

X für Sauerstoff oder Schwefel steht und

$R^5$ für Alkyl mit 1 bis 6 Kohlenstoffatomer, Halogenalkyl mit 1 bis 4 Kohlenstoffatomen und 1 bis 5 gleichen oder verschiedenen Halogenatomen, sowie für gegebenenfalls durch Fluor, Chlor, Brom, Methyl, tert.-Butyl, Methoxy, Methylthio, Trifluormethyl und/oder Trifluormethoxy substituiertes Phenyl oder Phenylalkyl mit 1 bis 2 Kohlenstoffatomen im Alkylteil steht.

Ganz besonders bevorzugt sind die Verbindungen der Formel (I), in welcher

$R^1$ und $R^2$ gleich oder verschieden sind und für Methyl, Ethyl Chlormethyl, Chlorethyl, Fluormethyl oder Fluorethyl stehen,

$R^3$ für Methyl, Ethyl, n-Popyl, n-Butyl, n-Pentyl, n-Hex-yl oder fur die Gruppierung $-(CH_2)_nR^4$ steht, in welcher

n für 0 oder 1 steht

$R^4$ für Chlormethyl, Fluormethyl,fur gegebenenfalls durch Fluor, Chlor, Brom, Methyl, Methoxy, Trifluormethyl oder Triflu-ormethoxy substituiertes Phenyl, sowie für die Gruppierung $XR^5$ steht,

in welcher

X für Sauerstoff oder Schwefel steht und

$R^5$ für Methyl, Ethyl, Trifluormethyl, Trichlormethyl, sowie für gegeöenenfalls durch Fluor, Chlor, Brom, Methyl, Methoxy, Trifluormethyl und/oder Trifluormethoxy suöstituiertes Phenyl oder Benzyl steht,

Hal für Chlor steht und

R für einen Rest $-CHR^6R^7$ steht,

in welchem

$R^6$ für Wasserstoff oder Cyano steht und

$R^7$ für 3-Phenoxy-phenyl, 4-Fluor-3-phenoxy-phenyl oder für Pentafluorphenyl steht.

In einer bevorzugten Variante (a) des Herstellungsverfahrens für die Verbindungen der Formel (I) werden 2,2-Dimethyl-3-(2-halogen-vinyl)-cyclopropancarbonsäure-chloride der Formel (IIa)

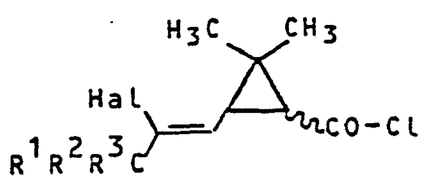

(IIa)

in welcher

$R^1$, $R^2$, $R^3$ und Hal die oben angegebenen Bedeutungen haben,

mit Alkoholen der Formel (III) (oben) in Gegenwart von Säureakzeptoren und unter Verwendung von Verdünnungsmitteln umgesetzt.

In einer weiteren bevorzugten Variante (b) - zur Herstellung von Verbindungen der Formel (I), in welcher $R^6$ für Cyano steht - werden 2,2-Dimethyl-3-(2-halogen-vinyl)-cyclopropancarbonsäurechloride der Formel (IIa) mit

α) entsprechenden Aldehyden der Formel (IV),

$R^7$ -CHO (IV)

in welcher

$R^7$ die oben angegebene Bedeutung hat,

und wenigstens der äquimolaren Menge eines Alkalicyanids (Natrium- oder Kaliumcyanid) in Gegenwart von Wasser und mit Wasser nicht mischbaren organischen Lösungsmitteln und gegebenenfalls in Gegenwart eines Katalysators umgesetzt; oder

β) Cyanhydrinen der Formel (V),

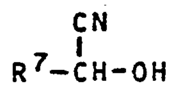 (V)

in welcher
$R^7$ die oben angegebene Bedeutung hat,
in Gegenwart von Säureakzeptoren und unter Verwendung von Verdünnungsmitteln umgesetzt.

Verwendet man als Ausgangsstoffe bei der Verfahrensvariante (a) beispielsweise 3-(2-Chlor-3,3-dimethyl-1-butenyl)-2,2-dimethyl-cyclopropancarbonsäurechlorid und 3-Phenoxy-benzylalkohol, so kann die Reaktion durch folgendes Formelschema skizziert werden:

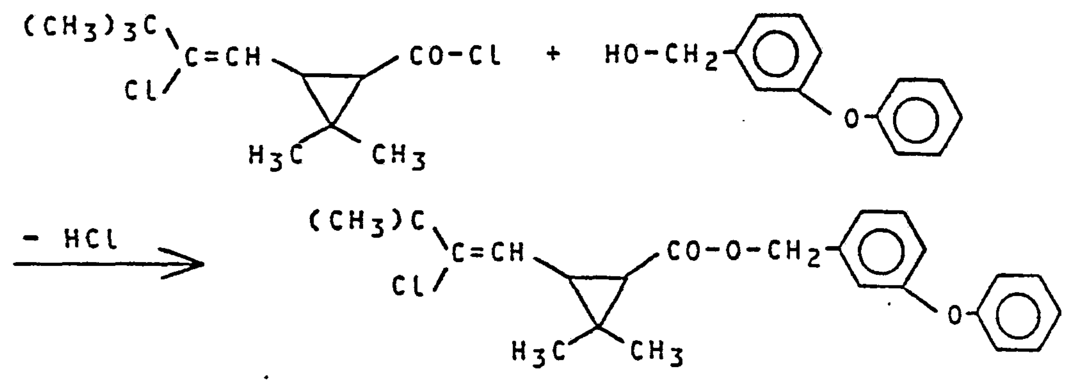


Verwendet man als Ausgangsstoffe bei der Verfahrensvariante (b/α) 3-(2-Chlor-3,3-dimethyl-1-butenyl)-2,2-dimethyl-cyclopropancarbonsäurechlorid, Natriumcyanid und 3-Phenoxy-benzaldehyd, so kann der Reaktionsablauf durch folgendes Formelschema skizziert werden:

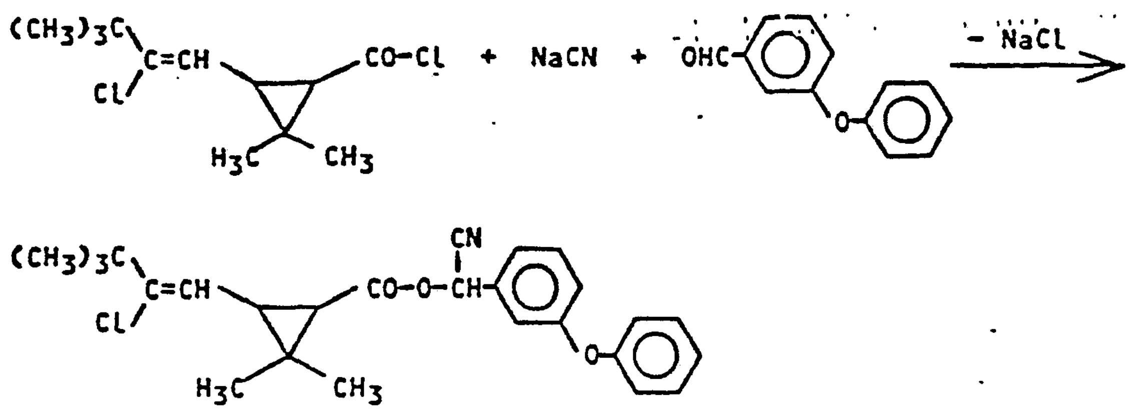


Verwendet man als Ausgangsstoffe bei der Verfahrensvariante (b/β) 3-(2-Chlor-3,3-dimethyl-1-butenyl)-2,2-dimethyl-cyclppropancarbonsäurechlorid und 3-Phenoxy-α-cyano-benzaldehyd, so kann der Reaktionsablauf durch folgendes Formelschema skizziert werden:

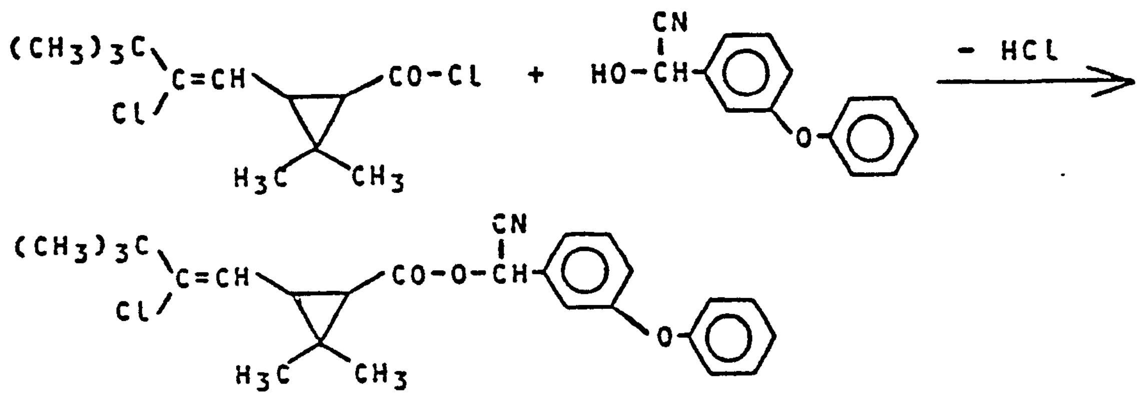


Die als Ausgangsstoffe zu verwendenden 2,2-Dimethyl-3-(2-halogen-vinyl)-cyclopropancarbonsäuren sind durch Formel (II), die entsprechenden Säurechloride durch Formel (IIa) definiert.

$R^1$, $R^2$, $R^3$ und Hal haben darin vorzugsweise bzw. insbesondere die gleichen Bedeutungen, wie sie bei der Definition des entsprechenden Restes in Formel (I) vorzugsweise bzw. als besonders bevorzugt angegeben sind.

Als Beispiele für die Verbindungen der Formel (II) und die entsprechenden Säurechloride der Formel (IIa) seien genannt:

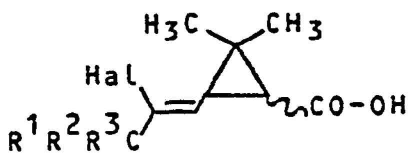 (II)

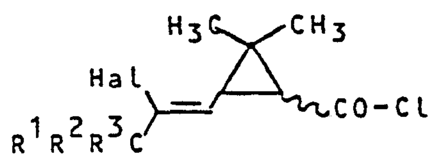 (IIa)

Hal = Chlor, Flour oder Brom

**Tabelle 1**

| $R^1$ | $R^2$ | $R^3$ |
|---|---|---|
| $CH_3$ | $CH_3$ | 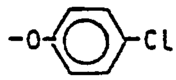 |
| $CH_3$ | $CH_3$ | 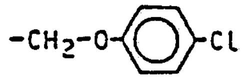 |
| $CH_3$ | $CH_3$ | 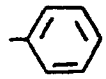 |
| $CH_3$ | $CH_3$ | $CH_3$ |
| $CH_3$ | $CH_3$ | $CH_2Cl$ |
| $CH_3$ | $CH_3$ | $CH_2F$ |
| $CH_3$ | $CH_3$ | 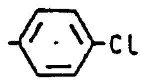 |
| $CH_3$ | $CH_3$ | $-SCF_3$ |
| $CH_3$ | $CH_3$ | 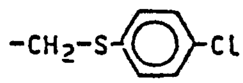 |
| $CH_3$ | $CH_3$ | $-CH_2OC_2H_5$ |
| $CH_3$ | $CH_3$ | $-SCCl_3$ |
| $CH_3$ | $CH_2Cl$ | $CH_3$ |
| $CH_3$ | $CH_2Cl$ | $CH_2Cl$ |
| $CH_3$ | $CH_2Cl$ | $CH_2F$ |
| $CH_3$ | $CH_2F$ | $CH_3$ |
| $CH_3$ | $CH_2F$ | $CH_2F$ |
| $CH_3$ | $CH_2F$ | $-SCF_3$ |
| $CH_3$ | $CH_3$ | 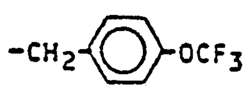 |
| $CH_3$ | $CH_3$ | 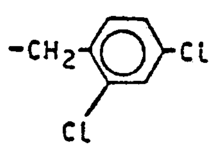 |
| $CH_3$ | $CH_3$ | $-CH_2OCH_3$ |
| $CH_3$ | $CH_3$ | 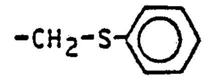 |
| $CH_3$ | $CH_3$ | 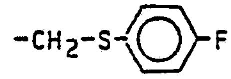 |
| $CH_3$ | $CH_3$ | 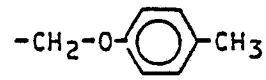 |
| $CH_3$ | $CH_3$ | 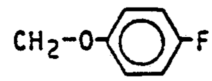 |
| $CH_3$ | $CH_3$ | 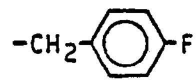 |
| $CH_3$ | $CH_3$ | 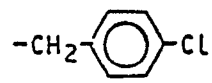 |
| $CH_3$ | $CH_3$ | 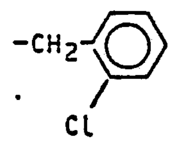 |
| $CH_3$ | $CH_3$ | 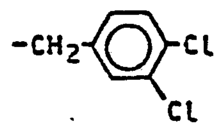 |
| $CH_3$ | $CH_3$ | $C_2H_5$ |
| $CH_3$ | $CH_3$ | $C_3H_7-n$ |
| $CH_3$ | $CH_3$ | $C_3H_7-i$ |
| $CH_3$ | $CH_3$ | $C_4H_9-n$ |
| $CH_3$ | $CH_3$ | $C_5H_{11}-n$ |
| $CH_3$ | $CH_3$ | $C_6H_{13}-n$ |
| $CH_3$ | $CH_3$ | $-CH_2-CH_2F$ |

Die 2,2-Dimethyl-3-(2-halogen-vinyl)-cyclopropancatbon-säurechloride der Formel (IIa) sind neu, sie können jedoch nach üblichen Methoden aus den entsprechenden Carbonsäuren der Formel (II) durch Umsetzung mit einem Chlorierungsmittel wie z.B. Thionylchlorid, gegebenenfalls unter Verwendung eines Verdunnungsmittels, wie z.B. Tetrachlorkohlenstoff, bei Temperaturen zwischen 10°C und 100°C hergestellt werden.

Die 2,2-Dimethyl-3-(2-halogen-vinyl)-cyclopropan-car-bonsäuren der Formel (II) sind ebenfalls neu. Man erhält sie z.B., wenn man Vinyl-Aldehyde der Formel (VI),

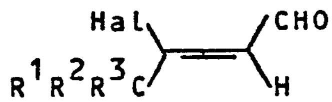 : (VI)

in welcher
$R^1$, $R^2$, $R^3$ und Hal die oben angegebenen Bedeutungen haben,
mit Methyl-butan-3-on der Formel (VII)

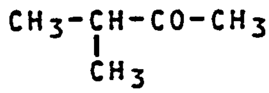 (VII)

in Anwesenheit von Halogenwasserstoffsäurenumsetzt, die dabei erhältlichen 4,4 Dimethyl-3-halogen-1-hexen-5-one der Formel (VIII),

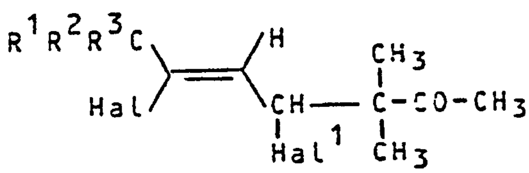 (VIII)

in welcher
$R^1$, $R^2$, $R^3$ und Hal die oben angegebenen Bedeutungen haben und
$Hal^1$ für Chlor oder Brom steht,
halogeniert und die dabei erhältlichen Verbindungen der Formel (IX),

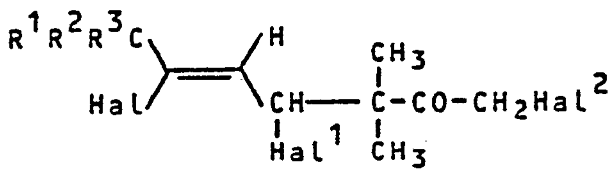 (IX)

in welcher
$R^1$, $R^2$, $R^3$, Hal und $Hal^1$ die oben angegebenen Bedeutungen haben und
$Hal^2$ für Chlor oder Brom steht,
mit Basen der Formel (X),
MO-Z (X)
in welcher
M für ein Aequivalent eines Alkali- oder Erdalkalimetallions steht und
Z für Wasserstoff oder $C_1$-$C_4$-Alkyl steht,
umsetzt.

Die als Ausgangsstoffe zu verwendenden Vinyl-Aldehyde sind durch die allgemeine Formel (VI) definiert. Vorzugsweise bzw. insbesondere bevorzugt stehen darin $R^1$ $R^2$, $R^3$ und Hal für diejenigen Reste, welche bereits bei der Definition von $R^1$, $R^2$, $R^3$ und Hal in Formel (I) vorzugsweise bzw. als insbesondere bevorzugt genannt wurden.

Die als Ausgangsstoffe zu verwendenden Vinyl-Aldehyde der Formel (VI) sind bekannt und/oder können

nach bekannten Methoden hergestellt werden (z.B. Zeitschrift für Chemie 1976 16, S. 337; 1973, 13, S. 97; Chem.Ber. 98, S. 3554).

Die außerdem als Ausgangsprodukte einzusetzende Verbindung 2-Methyl-butan-1-on der Formel (VII) ist bekannt.

Die Reaktion wird in Gegenwart mindestens äquimolarer Mengen Chlorwasserstoff oder Bromwasserstoff und gegebenenfalls unter Verwendung von Verdünnungsmitteln durchgeführt. Als Verdünnungsmittel kommen alle gegen Chloroder Bromwasserstoff inerten Lösungsmittel infrage. Bevorzugt wird ohne Lösungsmittel gearbeitet bei Temperaturen zwischen 0° C und 25° C.

Wird ohne Verdünnungsmittel gearbeitet, kann 2-Methyl-3-butanon im Ueberschun eingesetzt werden. Normalerweise werden 1 bis 10, vorzugsweise 1 bis 4 Aequivalente Keton auf ein Aequivalent Vinyl-Aldehyd der Formel (VI) verwendet.

Die bei der Umsetzung der Vinyl-Aldehyde der Formel (VI) mit dem Methylbutanon der Formel (VII) erhältlichen 4,4-Dimethyl-3-halogen-1-hexen-5-one der Formel (VIII) sind neu. Sie können isoliert und gereinigt oder ohne weitere Reinigung sofort in der nächsten Stufe weiter umgesetzt werden.

Verbindungen der Formel (IX) erhält man, indem man Verbindungen der Formel (VIII) (oben) halogeniert.

Die 4,4-Dimethyl-3-halogen-1-hexen-5-one sind durch die Formel (VIII) allgemein definiert. Vorzugsweise bzw. insbesondere bevorzugt stehen darin $R^1$, $R^2$, $R^3$ und Hal für diejenigen Reste, welche bereits bei der Definition von $R^1$, $R^2$, $R^3$ und Hal in Formel (I) vorzugsweise bzw. als insbesondere bevorzugt genannt wurden. Hal steht in dieser Formel für Chlor oder Brom.

Als Halogenierungsmittel kommen Chlor, Brom oder Sulfurylchlorid infrage.

Es wird üblicherweise in einem inerten Verdünnungsmittel gearbeitet. Als solche dienen beispielsweise Chlorkohlenwasserstoffe, wie z.B. Chloroform.

Die Reaktionstemperatur sollte 40° C nicht überschreiten, vorzugsweise wird zwischen -l0° C und +250C gearbeitet.

Es wird normalerweise ein Aequivalent Halogenierungsmittel pro Mol Verbindung der Formel VIII eingesetzt. Es kann jedoch auch ein Überschuß an Verbindungen der Formel VIII verwendet werden.

Die bei der Halogenierung der Verbindungen der Formel (VIII) erhältlichen Verbindungen der Formel (IX) sind neu. Sie können isoliert und gereinigt oder ohne weitere Reinigung sofort in der nächsten Stufe weiter umgesetzt werden.

Die 2,2-Dimethyl-3-(2-halogen-vinyl)-cyclopropancarbon-säuren der Formel (II) (oben) erhält man, wenn man die Verbindungen der Formel (IX) mit Basen der Formel (X) umsetzt.

Die beim Verfahren zu verwendenden Ausgangsstoffe sind durch die Formeln (IX) und (X) allgemein definiert.

Vorzugsweise bzw. insbesondere bevorzugt stehen darin $R^1$, $R^2$, $R^3$ und Hal für diejenigen Reste, welche bereits bei der Definition von $R^1$, $R^2$, $R^3$ und Hal in Formel (I) vorzugsweise bzw. als insbesondere bevorzugt genannt wurden. $Hal^1$ und $Hal^2$ stehen für Chlor oder Brom, M steht für ein Moläquivalent eines Alkali- oder Erdalkali-metallions und Z für Wasserstoff oder $C_1$-$C_4$-Alkyl.

Im einzelnen seien als Beispiele für Basen genannt : Natriumhydroxid, Kaliumhydroxid, Calciumhydroxid, Natriummethylat, Natriumethylat, Natriumbutylat, Kaliumtert.-butylat.

Bevorzugt wird bei Verwendung der Hydroxide in Wasser und/oder einem inerten Verdünnungsmittel gearbeitet. Hier kommen beispielsweise Alkohole wie Methanol, Ethanol, t-Butanol, Ether wie Dioxan, Tetrahydrofuran, Dimethoxyethan oder Ketone wie Aceton sowie Dimethylformamid infrage. Es können jedoch auch nicht mit Wasser mischbare Lösungsmittel, wie Methylenchlorid, Petrolether, Cyclohexan, Toluol oder Chlorbenzol, gegebenenfalls in Gegenwart eines Phasentransferkatalysators, verwendet werden.

Bei Verwendung der Alkoholate wird am günstigsten in den entsprechenden Alkoholen gearbeitet.

Die Reaktionstemperaturen können in einem größeren Bereich variiert werden. Im allgemeinen arbeitet man zwischen 0° C und 150° C, vorzugsweise zwischen 20° C und 100oC

Es müssen mindestens 2 Aequivalente Basen (X) auf ein Mol Ausgangsstoff der Formel (IX) eingesetzt werden. Ein Ueberschuß an Base bis zu 10 Aequivalenten ist meist vorteilhaft. Die Aufarbeitung des Reaktionsgemisches erfolgt bei Herstellung der Säuren (X = H) durch Extraktion im Alkalischen (zur Entfernung von Verunreinigungen) und nach Ansäuern der Wasserphase durch erneute Extraktion. Bei Herstellung von Estern erfolgt die Reinigung durch Destillation. Vorher wird mit Wasser verdünnt, neutral gestellt und extrahiert (vgl. hierzu die Herstellungsbeispiele).

Die weiter als Ausgangsstoffe - für die Herstellung der Verbindungen der Formel (I) - zu verwendenden Alkohole und Cyanhydrine sind durch die allgemeine Formeln (III) und (V) definiert. In diesen Formeln steht R und $R^7$ vorzugsweise bzw. insbesondere für diejenigen Reste, welche bereits bei der Definition von R und $R^7$ in Formel (I) vorzugsweise bzw. als insbesondere bevorzugt genannt wurden.

Als Beispiele für die Alkohole der Formel (III) und Cyanhydrine der Formel (V) seien genannt 3-Phenoxybenzylalkohol, 3-Phenoxy-4-fluor-benzylalkohol, Pentafluorbenzylalkohol, α-Cyano-3-phenoxy-benzylalkohold α-Cyano-3-phenoxy-4-fluor-benzylalkohol.

Die Verbindungen der Formel (III) sind bereits bekannt (vgl. US-PS 41 63 787, US-PS 38 35 176, DE-OS 27 09 264, DE-OS 31 03 325, DE-OS 27 39 854, DE-OS 26 58 074 und J.Chem.Soc. 1961, 808-817).

Die als Ausgangsstoffe verwendbaren Aldehyde sind durch die Formel (IV) definiert. $R^7$ hat darin die gleichebevorzugte bzw.besonders bevorzugte Bedeutung wie die entsprechenden Reste in Formel (I).

Als Beispiele für die Aldehyde der Formel (IV) seien genannt: 4-Fluor-3-phenoxy-benzaldehyd, 3-Phenoxy-

benzaldehyd, α-Cyano-4-fluor-3-phenoxy-benzaldehyd, α-Cyano-3-phenoxy-benz-aldehyd.

Die Verbindungen der Formel (IV) sind bereits bekannt (vgl. US-PS 41 63 787, US-PS 38 35- 176, DE-OS 27 09 264, D-E-OS 31 03 325 DE-OS 27 39 854 und DE-OS 26 58 074).

Das Verfahren zur Herstellung der neuen Verbindungen der Formel (I) wird in allen Varianten vorzugsweise unter Verwendung von Verdünnungsmitteln durchgeführt. Als Verdünnungsmittel kommen praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether,Tetrahydrofuran und Dioxan, Ketone, wie Aceton, Methylethyl-, Methyl-isopropyl- und Methyl-iso-butyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Varianten (a) und (b/β) des erfindungsgemänen Verfahrens werden vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und Kaliumcarbonat, Natrium- und Kaliumethylat bzw. -methylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die Variante (b/α) des erfindungsgemäßen Verfahrens wird in Gegenwart von Wasser und eines der oben genannten organischen Lösungsmittel, soweit mit Wasser nicht-mischbar, durchgeführt. Hierfür sind insbesondere die oben genannten Kohlenwasserstoffe geeignet.

Als Katalysatoren werden bei der Verfahrensvariante (b/α) vorzugsweise Verbindungen verwendet, welche zum Transfer von Anionen aus Wasser in organische Lösungsmittel geeignet sind. Beispiele hierfür sind Benzyl-triethylammonium-hydrogensulfat, Tetrabutylammonium-bromid und Methyl-tri-octyl-ammonium-chlorid (Aliquat 336®).

In allen Verfahrensvarianten kann die Reaktionstemperatur innerhalb eines gröneren Bereiches variiert werden. Im allgemeinen arbeitet man zwischen 0°C und 100°C, vorzugsweise bei 10°C bis 50°C.

Das erfindungsgemäße Verfahren wird im allgemeinen bei Normaldruck durchgeführt. Zur Durchführung des erfindungsgemäßen Verfahrens werden die Ausgangsstoffe gewöhnlich in äquimolaren Mengen eingesetzt. Ein Ueberschuß der einen oder anderen Reaktionskomponente bringt keine wesentlichen Vorteile. Die Ausgangsstoffe werden in geeigneten Verdünnungsmitteln zusammengegeben und gegebenenfalls nach Zugabe eines Säureakzeptors und/oder eines Katalysators - bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z.B. Toluol, verdünnt, die organische Phase abtrennt, mit Wasser wäscht, trocknet, filtriert und vom Filtrat das Lösungsmittel unter vermindertem Druck und bei mäßig erhöhter Temperatur sorgfältig abdestilliert ('andestillieren').

Die 2,2-Dimethyl-3-(2-halogen-vinyl)-cyclopropancarbon-säureester zeichnen sich, wie bereits erwähnt, durch hohe insektizide und akarizide Wirksamkeit aus.

Sie können gegen pflanzenschädigende Insekten und Milben in Land- und Forstwirtschaft sowie gegen Ektoparasiten im veterinärmedizinischen Bereich eingesetzt werden.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidim vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoolus differentialis, Schistocerceca gregaria.

Aus der Ordnung der Dermaptera z.B. Forficula auricularia.

Aus der Ordnung der Isoptera z.B. Reticulitermes spp..

Aus der Ordnung der Anoplura z.a. Phylloxera vastatrix, Pemphigus spp., Pediculus humenus corporis, Haematöpinus spp., Linognathus spp.

Aus der Ordnung der Mallophaga z.B..Trichodectes spp., Damalinea spp.

Aus der Ordnung der Thysanoptera z.B. Hercinothrips femoralis, Thrips tabaci.

Aus der Ordnung der Heteroptera z.B. Eurygaste spp., Dysdercus intermedius, Piesma qusdrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis

gossypii, Brevicoryne brassicae, Cryptomyzus ribis, Doralis fabae, Doralis pomi, Eriosoma lanigerum, Hyalopterus arundinis, Macrosiphum avenae, Myzus spp., phoredon humuli, Rhopalosiphum padi, Empoasca ssp., Euscelis bilobatus, Nephoettix cincticeps, Lecanium corni, Saissetia cleae, Lacdelphax striatellus, Nilaparvata lugens, Acnidiella aurantii, Aspidiotus hederae, Pseudococcus spp. Psylla spp. Aus der Ordnung der Lepidoptera z.B. Pectinophora cossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyliocnistis citrella, Agiotis spp., Euxoa spp., Feitia spp., Earias insulana, Heliothis spp., Laphygma exigua, Mamestra brassicae, Panolis flammea, Prodenia litura, Spodoptera spp., Trichoplusiani, Carpocapsa pomohella, Pieris spp., Chilo spp., Pyrausta nubilalis, Ephestia kuehniella, Galleria mellonella, Tineola bisselliella, Tinea pellionella, Hofmannphila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides Obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Antohonomus spp., Sitophilus spp., Otiorrhynchus sulcatus, Cosmopolites soridus, Ceuthorrhynchus assimilis, Hypera postica Dermestes spp., Trogoderma spp., Anthrenus spp., Attagenus spp., Lyctus spp., Meligethes aeneus, Ptinus spp., Niptus hololeucis Gibbium psylloides, Tribollum spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharacnis, Vespa spp. Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllccoptruta oleivora, Boophilus spp., Rhipicephalus spp., Amblyomma spp., Hyalomma spp., Ixcdes spp., Psoroptes spp., Chorioptes spp., Sarcoptes spp., Tarsonemus spp., Bryobia praetiosa, Panonycmus spp., Tetranychus spp..

Die neuen Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalinc, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid als feste Trägerstoffe kommen in Frage: z.B. natürliche Gesteinsmehle, wie Kaoline, Tonercen, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und frak.tiorrierte natürliche Gesteine wie Calcit, Marmor, Bims, Sepiolith, Dolomit sowie syntnetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Oispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige- Polymere verwendet werden, wie Gurmmiarabicum, Polyvinylalkohol, Polyvimylacetat.

Es können Farbstoffe wie anorganische Picmente, z.B. Eisenoxid, Titanoxid, Ferrocyanblau und organische Farbstoffe, wie Alizarin-, Azou Metallphthalocyaninfarbstoffe und Spurennährstoffe wie Salze von Eisen, Mangan, Bor, Xupfer, Kobalt, Molybdän und Zink verwendet werden.

Die Formulierungen enthalten im allgemeinen zwischen 0,1 und 95 Gewichtsprozent Wirkstoff, vorzugsweise

zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilentien, Aksriziden, Nemstiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorgenismen hergestellte Stoffe u.a.

Die erfindungsgemäßen Wirkstoffe können ferner in ihren handelsüblichen Formulierungen sowie in den aus diesen Formulierungen hereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsüeise zwischen 0,0001 und 1 Gew.-% liegen.

Die Anwendung geschieht in einer den Anwendungsformen angepeßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf geüälkten Unterlagen aus.

Die erfindungsgemäßen Wirkstoffe eigenen sich auch zur Bekämpfung von Ekto-und Endoparasiten auf dem Gebiet der Tierhaltung und Viehzucht, wobei durch die Bekämpfung der Schädlinge beesere Ergebnisse, z,B. höhere Milchleistungen, höhere Gewicht, schöneres Tierfell, längere Lebensdauer usw. erreicht werden können.

Die Anwendung der erfindungsgemäßen Wirkstoffe geschieht auf diesen Gebieten in bekannter Weise, wie durch äußerliche Anwendung in Form beispielsweise des Tauchens (Dippen), Sprühens (Sprayen), Aufgießens (pour-on and spot-on) und des Einpuderns sowie durch orale Anwendung, beispielsweise über das Futter oder Trinkwasser in Form von beispielsweise Tabletten, Kapseln, Tränken, Granulaten.

**Herstellungsbeispiele:**

**Beispiel 1**

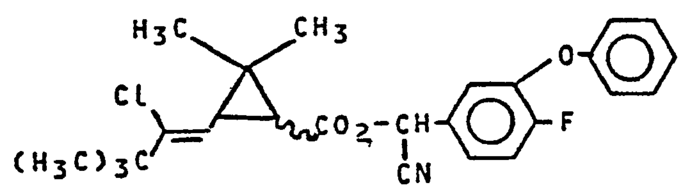

17,6 g (0,07 Mol) cis/trans-3-(Z-2-Chlor-,3-dimethyl-1-butenyl)-2,2-dimethyl-cyclopropan-1-carbonsäurechlorid und 17,17 g (0,D7 Mol) α-Cyano-4-fluor-3-phenoxy-benzylalkohol werden in 300 ml Toluol vorgelegt. Dann wird bei 20°C 5,58 g (0,07 Mol) Pyridin zugetropft und 16 Stunden nachgerührt. Anschließend wird mit Wasser, Natriumhydrogencarbonatlösung und Wasser gewaschen. Die organische Phase wird abgetrennt, über Natriumsulfat getrocknet und eingeengt. Die letzten Lösungsmittelreste werden durch 'Andestillieren' entfernt.

Man erhält 31,8 g (99,7 % der Theorie) cis/trans-3-(Z-2-Chlor-3,3-dimethyl-1-butenyl)-2,2-dimethyl-cyclopropan-1-carbonsäure-α-cyano-4-fluor-3-phenoxy-benzyl-ester als nahezu farbloses Oel mit einem Brechungsindex $n_D^{20}$: 1,539.

Analog Beispiel 1 bzw. gemäß Verfahrenvatianten (a), (b/α) und b/β) werden die übrigen Verbindungen der Formel (I) erhalten:

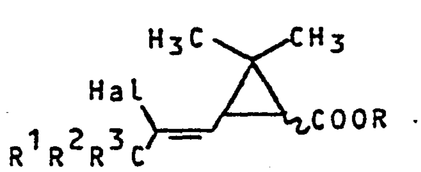

(I)

**Tabelle 2**

| Beisp. Nr. | Formel | Brechungsindex $n_D^{20}$ |
|---|---|---|
| 2 | 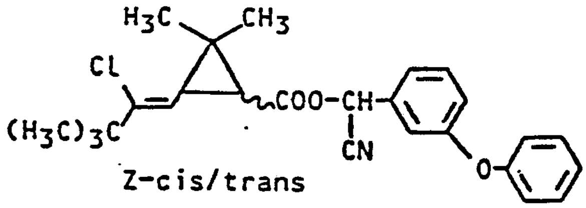 Z-cis/trans | 1,545 |
| 3 | 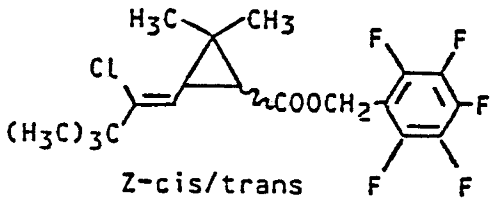 Z-cis/trans | 1,479 |
| 4 | 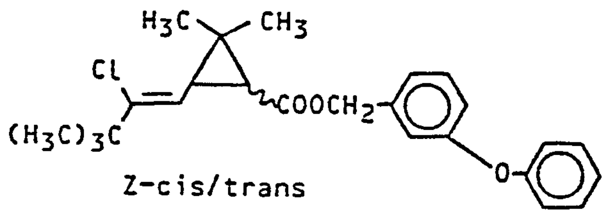 Z-cis/trans | |
| 5 | 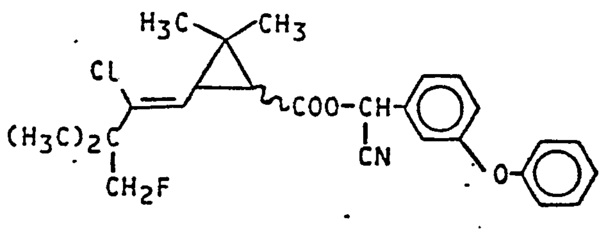 Z-cis/trans | 1,542 |

**Tabelle 2 - Forsetzung**

| Beisp. Nr. | Formel | Brechungsindex $n_D^{20}$ |
|---|---|---|
| 6 | 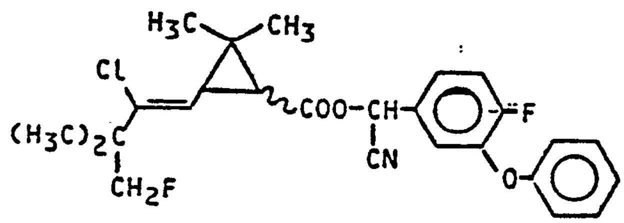<br>Z-cis/trans | 1,535 |
| 7 | 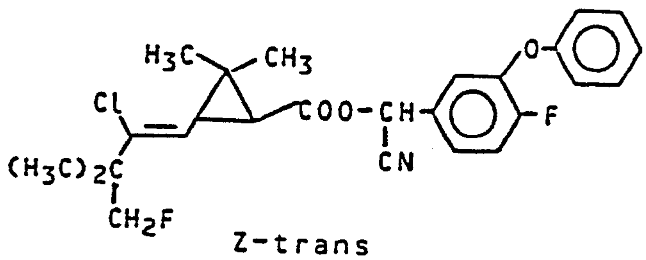<br>Z-trans | 1,536 |
| 8 | 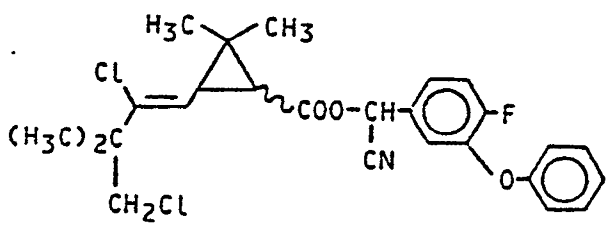<br>Z-cis/trans | 1,535 |
| 9 | 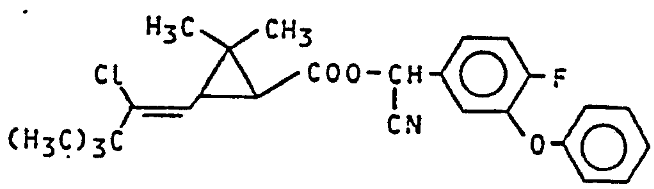<br>Z-trans | 1,536 |

**Herstellung der Ausgangsprodukte der Formel (VI)**

**Beispiel (VI-1)**

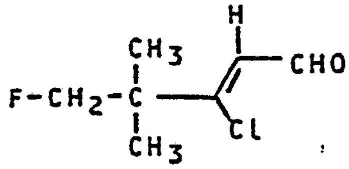

Zu 175,2 g (2,4 Mol) Dimethylformamid werden bei ca. 10°C 307 g (2 Mol) Phosphoroxychlorid getropft und anschließend werden bei 20°C 94,4 g (0,8 Mol) 4-Fluor-3,3-dimethyl-2-butanon zugetropft. Die Temperatur steigt nach 30 Minuten langsam auf 50°C an. Nach 3-stündigem Nachrühren wird auf 2,5 1 Eiswasser gegossen- und dreimal mit Ether extrahiert. Die Wasserphase wird mit Natriumacetat auf pH 5,6 gestellt und erneut dreimal mit Ether extrahiert. Diese Extrakte werden mit Natriumhydrogencarbonatlösung und dann mit Wasser gewaschen, über Natriumsulfat getrocknet und eingeengt.

Nach Destillation erhält man 68,8 g 5-Fluor-3-chlor-4,4-dimethyl-2-penten-1-al vom Siedepunkt 89°C bis 91°C/ 14 mbar.

**Beispiel (VI-2)**

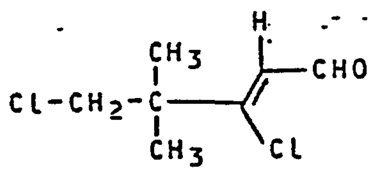

Zu 157,7 g (2,16 Mol) Dimethylformamid werden bei ca. 10°C 276,5 g (1,8 Mol) Phosphoroxychlorid zugetropft und anschließend werden bei 20°C 97,5 g (0,72 Mol) 4-Chlor-3,3-dimethyl-2-butanon zugetropft. Nach beendetem Zutropfen wird nicht mehr gekühlt, so daß die Temperatur auf ca. 35°C steigt. Nach 3-stündigem Nachrühren wird auf 2 1 Eiswasser gegossen. Die Aufarbeitung erfolgt wie in Beispiel (VI-1).

Man erhält 23,1 g 3,5-Dichlor-4,4-dimethyl-2-penten-1-al vom Siedepunkt 112°C/14 mbar.

Analog Beispiel (VI-1) und (VI-2) werden die übrigen Ausgangsstoffe der Formel (VI) erhalten.

**Herstellung der Ausgangsprodukte der Formel (VIII**

**Beispiel (VIII-1)**

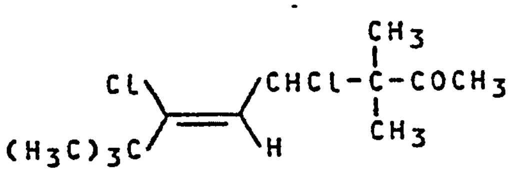

Eine Mischung aus 29,3 g (0,2 Mol) 3-Chlor-4,4-dimethyl-2-pentenal und 34,4 g (0,4 Mol) Methylisopropylketon wird bei 10°C mit Chlorwasserstoff gesättigt und 12 Stunden bei 20°C nachgerührt. Die Lösung wird mit Wasser gewaschen, mit Natriumcarbonat neutralisiert und dreimal mit Methylenchlorid extrahiert. Die vereinigten organischen Phasen werden mit Wasser gewaschen, über Natriumsulfat getrocknet und eingeengt.

Man erhält 45,4 g (90,4 % der Theorie) 4,6-Dichlor-3,3, 7,7-tetramethyl-5-octen-2-on vom Siedepunkt Kp. 120°C/ 0,1 mbar.

Analog Beispiel (VIII-1) werden die übrigen Verbindungen der Formel (VIII) erhalten wie z.B.

**Beispiel (VIII-2)**

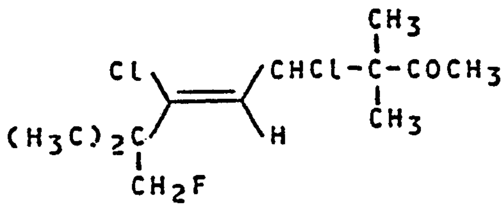

Kp. 110°C/ 0,1 mbar

**Beispiel (VIII-3)**

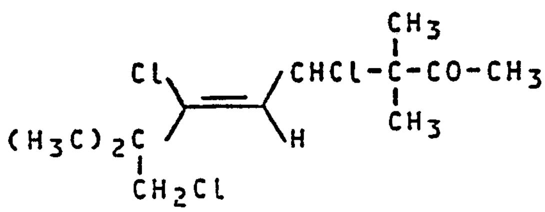

Kp. 130°C/ 0,1 mbar

**Ausgangsprodukte der Formel (IX)**

**Beispiel (IX-1)**

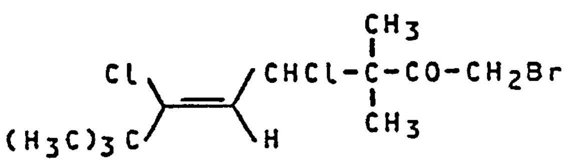

20 g (0,0796 Mol) 4,6-Dichlor-3,3,7,7-tetramethyl-5-octen-2-on werden in 350 ml Chloroform gelöst und bei 20°C bis 25°C mit 12,/4 g (0,0/Y6 Mol) brom verserzr. Nach ca. 15 Minuten wird Bromwasserstoff entwickelt. Die Lösung entfärbt sich nach ca. 45 Minuten und wird eingeengt.

Man erhält 26,3 g rohes 1-Brom-4,6-dichlor-3,3,7,7-tetramethyl-5-octen-2-on, dessen Struktur durch das $^1$H-NMR-Spektrum gesichert wird. Es wird direkt in die nächste Stufe eingesetzt.

Analog Beispiel (IX-1) werden die übrigen Verbindungen der Formel (IX) erhalten, wie z.B.:

**Beispiel (IX-2)**

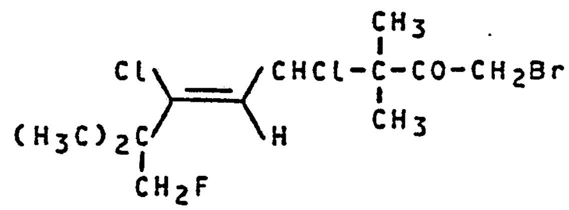

**Beispiel (IX-3)**

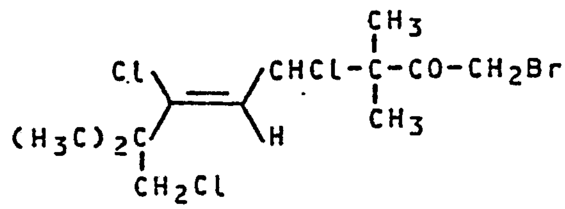

**Ausgangsprodukte der Formel (II)**

**Beispiel (II-1)**

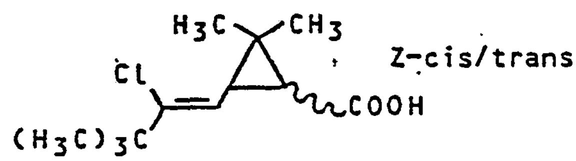

Z-cis/trans

Zu einer Mischung aus 31,75 g (0,793 Mol) Natriumhydroxid in 286 ml Wasser und 30 ml Dioxan werden 26,2 g 1-Brom-4,6-dichlor-3,3,7,7-tetramethyl-5-octen-2-on in 50 ml Dioxdn zugetropft. Bei 20°C wird 10 Stunden nachgerührt, mit Wasser verdünnt und dreimal mit Methylenchlorid extrahiert. Die wässrige Phase wird mit konzentrierter Salzsäure angesäuert und erneut dreimal mit Methylenchlorid extrahiert.

Die sauren Extrakte werden vereinigt, über Natriumsulfat getrocknet und eingedampft.

Die 3-(Z-2-Chlor-3,3-dimethyl-1-butenyl)-2,2-dimethyl-cyclopropan-1-carbonsäure bleibt als Oel zurück (cis trans-Gemisch ca. 35 : 65), das langsam zu kristallisieren beginnt.

**Beispiel (II-2)**

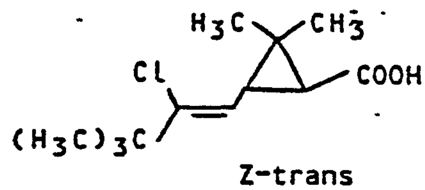

Z-trans

Zu einer Lösung aus 88 %-igem gepulvertem, technischem Kaliumhydroxid in Methanol (60,6 ml 3 N-Lösung) werden bei 50°C 10 g rohes 1-Brom-4,6-dichlor-3,3,7,7-tetra-methyl-5-octen-2-on (Beispiel (IX-1)) zugetropft. Nach 15 Minuten wird abgekühlt und noch 8 Stunden bei 20°C nachgerührt. Anschließend wird mit Wasser verdünnt und dreimal mit Methylenchlorid extrahiert. Die Wasserphase wird angesäuert mit konzentrierter Salzsäure und erneut dreimal mit Methylenchlorid extrahiert. Die organischen Phasen werden vereinigt, über Natriumsulfat getrocknet und eingeengt.

Nach Verreiben mit Petrolether erhält man trans-3-(Z-2-Chlor-3,3-dimethyl-1-butenyl)-2,2-dimethyl-cyclopropan-1-carbonsäure vom Schmelzpunkt Fp.: 135°C.

Analog Beispiel (II-1) und (II-2) werden die übrigen Verbindungen der Formel (II) hergestellt, wie z.B.:

**Beispiel (II-3)**

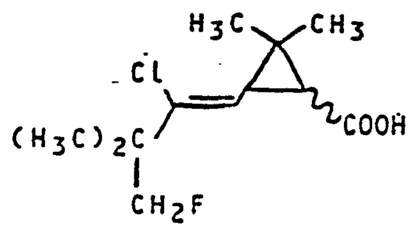

Z-cis/trans

Fp. 97-115°C

**Beispiel (II-4)**

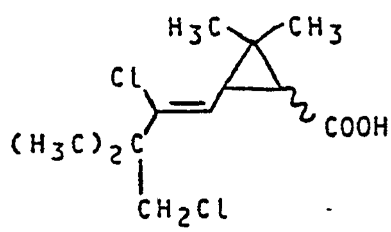

Z-cis/trans

Fp. 78-84°C

**Beispiel (II-5)**

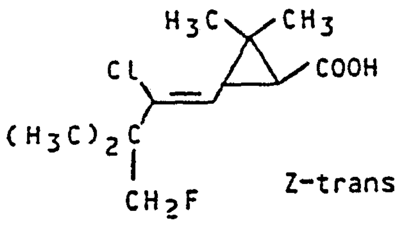

Z-trans

Fp. 141°C

**Ausgangsprodukte der Formel (IIa)**

**Beispiel (IIa-1)**

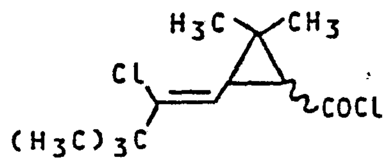

Z-cis/trans

38,9 g (0,168 Mol) cis/trans-3-(Z-2-Chlor-3,3-dimethyl-1-butenyl)-2,2-dimethyl-cyclopropan-1-carbonsäure werden in 380 ml Tetrachlorkohlenstoff gelöst und mit 30,2 g (0,253 Mol) Thionylchlorid tropfenweise versetzt. Anschließend wird 4 Stunden unter Rückfluß erhitzt und eingeengt.

Man erhält 45,4 g Rohprodukt, das durch Destillation im Hochvakuum (Kugelrohr) gereinigt wird.

Die Ausbeute beträgt 36,25 g (75,5 % der Theorie) cis/-trans-3-(Z-2-Chlor-3,3-dimethyl-1-butenyl)-2,2-dimethyl-cyclopropan-1-carbonsäurechlorid mit einem Siedepunkt von Kp.: 90°C/0,1 mbar.

Analog Beispiel (IIa-1) werden die übrigen Verbindungen der Formel (IIa) erhalten.

**Beispiel A**

Heliothis armigera - Test

Lösungsmittel: 3 Gewichtsteila Aceton

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungzmittel und der angegebenen Menge Emulgator und verdünnt das Konzentrat mit Wasser auf die gewünschte Konzentration.

Sojatriebe (Glycine max) werden durch Tauchen in die Wirkstoffzubeteitung der gewünschten Konzentration behandelt und mit der Tabakknospenraupe (Heliothis armigera) besetzt, solange die Blätter noch feucht sind.

Nach der gewünschten Zeit wird die Abtötung in % bestimmt. Dabei bedeutet 100 7, daß alle Raupen abgetötet 0 % bedeutet, daß keine Raupen abgetötet wurden.

Bei diesem Test zeigen z.B. die folgenden Verbindungen der Herstellungsbeispiele überlegene Wirksamkeit gegenüber Stand der Technik: (1) und (2).

**Beispiel B**

Tetranychus-Test (resistent)
Lösungsmittel: 3 Gewichtzteile Aceton
Emulgator: 1 Gewichtsteil Alkylarylpolyglykoläther

Zur Herstellunf einer zweckmäßigen Wirkstoffzubereitung vermischt man 1 Gewichtsteil Wirkstoff mit der angegebenen Henge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in a bestimmt. Dabei bedeutet 100 %, daß alle Spinnmilben abgetötet wurden. 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: (1), (6), (7) und (9).

**Beispiel C**

Grenzkonzentrations-Test /Bodeninsekten
Testinsekt: Phorbia antiqua-Maden im Boden
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und dach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Tetinsekten leben wie bei der unbehandelten Kontrolle.

Bei diesem Test zeigen z.B. die folgenden Verbindung der Herstellungs. beispiele überlegene Wirkung gegenüber dem Stand der Technik: (1), (2), (5), (6) und (8).

**Beispiel D**

Test mit Boophilus microplus resistent
Lösungsmittel: 35 Gewichtsteile Ethylenglykolmonomethylether
35 Gewichtsteile Nonylphenolpolylglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erheltene Konzentrat mit Wasser auf die gewünschte Konzentration.

10 adulte Boophilus microplus res. werden in die zu testende Wirkstoffzubereitung 1 Minute getaucht. Nach Ueberführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: (1), (2), (5), (5) und (9).

**Beispiel E**

Test mit Lucilia cuprina res.-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser suf die jeweils gewünschte Konzentration.

Etwa 20 Lucilia cuprina res.-Larven werden in ein Teströhrchen gebracht, welches ca. 1 $cm^2$ Pferdefleisch

und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: (1), (2) und (9).

**Patentansprüche**

1. 2,2-Dimethyl-3-(2-halogen-vinyl)-cyclopropan-carbonsäureester der Formel

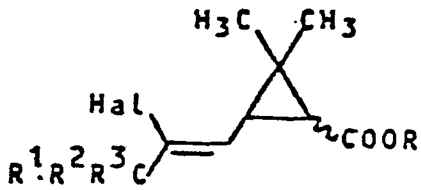

(I)

in welcher

$R^1$ und $R^2$ gleich oder verschieden sind und für Alkyl oder Halogenalkyl stehen,

$R^3$ für Alkyl oder für die Gruppierung $-(CH_2)_nR^4$ steht,

in welcher

$R^4$ für Halogenalkyl, für gegebenenfalls durch Fluor, Chlor, Brom, Methyl, tert.-Butyl, Methoxy, Methylthio, Trifluormethyl und/oder Trifluormethoxy substituiertes Phenyl öder fur die Gruppierung $-XR^5$ steht

in welcher

X für Sauerstoff oder Schwefel steht und

$R^5$ für Alkyl, Halogenalkyl, für gegebenenfalls durch Fluor, Chlor, Brom, Methyl, tert.-Butyl, Methoxy, Methylthio, Trifluormethyl und/oder Trifluormethoxy substituiertes Phenyl oder Phenylalkyl steht

Hal für Fluor, Chlor oder Brom steht und

R für einen Rest $-CHR^6R^7$ steht, in welchem $R^6$ für Wasserstoff oder CN steht und $R^7$ für 3-Phenoxy-phenyl, 4-Fluor-3-phenoxyphenyl oder für Pentafluorphenyl steht.

2. Verfahren zur Herstellung von Verbindungen der Formel (I), in welcher

$R^1$ und $R^2$ gleich oder verschieden sind und für Alkyl oder Halogenalkyl stehen,

$R^3$ für Alkyl oder für die Gruppierung $-(CH_2)_nR^4$ steht, in welcher

n für 0 oder 1 steht,

$R^4$ für Halogenalkyl, für gegebenenfalls durch Fluor, Chlor, Brom, Methyl, tert.-Butyl, Methoxy, Methylthio, Trifluormethoxy und/oder Triflourmethyl substituiertes Phenyl oder für die Gruppierung $-XR^5$ steht, in welcher

X für Sauerstoff oder Schwefel steht und

$R^5$ für Alkyl, Halogenalkyl, für gegebenenfalls durch Fluor, Chlor, Brom, Methyl, tert.-Butyl, Methoxy, Methylthio, Trifluormethyl und/oder Trifluormethoxy substituiertes Phenylalkyl steht,

Hal für Fluor, Chlor oder Brom steht und

R für einen Rest $-CHR^6R^7$ steht, in welchem $R^6$ fur Wasserstoff oder CN steht und $R^7$ für 3-Phenoxy-phenyl, 4-Fluor-3-phenoxypheny oder für Pentafluorphenyl steht,

dadurch gekennzeichnet, daß man

2,2-Dimethyl-3-(2-halogen-vinyl)-cyclopropancarbon-säuren der Formel (II)

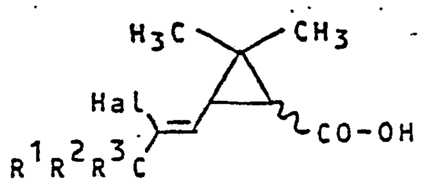

(II)

in welcher

$R^1$, $R^2$, $R^3$ und Hal die oben angegebenen Bedeutungen haben,

oder reaktionsfähige Derivate derselben mit Alkoholen der Formel

R-OH (III)

in welcher

R die oben angegebene Bedeutung hat,

oder mit reaktionsfähigen Derivaten derselben gegebenenfalls in Gegenwart von Säureakzeptoren, gegebenenfalls in Gegenwart von Katalysatoren und gegebenenfalls in Gegenwart von Verdünnungsmitteln umsetzt.

Verbindungen der Formel (I) gemäß Anspruch 1, in welcher
$R^1$ und $R^2$ gleich oder verschieden sind und für Methyl, Ethyl, Chlormethyl, Chlorethyl, Fluormethyl oder Fluorethyl stehen,
$R^3$ für Methyl, Ethyl, n-Propyl, i-Propyl, n-Butyl, n-Pentyl, n-Hexyl oder für die Gruppierung $-(CH2)_nR^4$ steht,in welcher
n für 0 oder 1 steht,
$R^4$ für Chlormethyl, Fluormerhyl,für gegebenenfalls durch Fluor, Chlor, Brom, Methyl, Methoxy, Trifluormethyl oder Trifluormethoxy substituiertes Phenyl, sovie für die Gruppierung $XR^5$ steht,
in welcher
X für Sauerstoff oder Schwefel steht und
$R^5$ für Methyl, Sthyl, Trifluormethyl, Trichlormethyl, soxie für gegebenenfalls durch Fluor, Chlor, Srom, Methyl,-Methoxy, Irifluormethyl und/oder Trifluormethoxy substituiertes Phenyl oder Benzyl steht, und
Hal für Chlor steht.

4. 2,2-Dimethyl-3-(2-halogenvinyl)-cycloDropan-carbon-säuren und ihrer Derivate der Formel II b

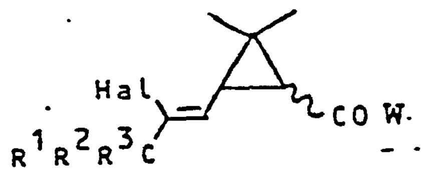

II b

in welcher
$R^1$, $R^2$, $R^3$ und Hal die in Anspruch 1 angegebene Bedeutung haben und
W für OH, Halogen, $C_{1-4}$-Alkoxy steht.

5. Verfahren zur Herstellung der 2,2-Dimethyl-3-(2-halogen-vinyl)-cyclopropan-carbonsäuren und ihrer Derivate der Formel (II b), dadurch gekennzeichnet, daß man Vinyl-Aldehyde der Formel (VI),

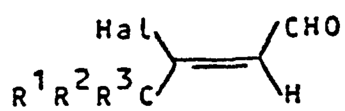

(VI)

in welcher
$R^1$, $R^2$, $R^3$ und Hal die in Anspruch 1 angegebenen Bedeutungen haben,
mit Methyl-butan-3-on der Formel (VII)

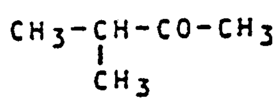

(VII)

in Anwesenheit von Halogenwasserstoffsäuren umsetzt, die dabei erhältlichen 4,4-Dimethyl-3-halogen-1-hexen-5-one der Formel (VIII),

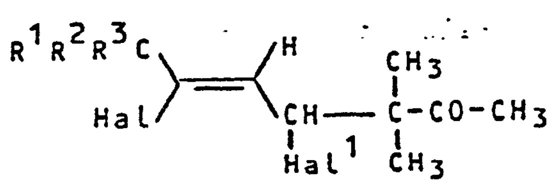

(VIII)

in welcher
$R^1$, $R^2$ $R^3$
und Hal die oben angegebenen Bedeutungen haben und
$Hal^1$ für Chlor oder Brom steht,
halogeniert und die dabei erhältlichen Verbindungen der Formel (IX),

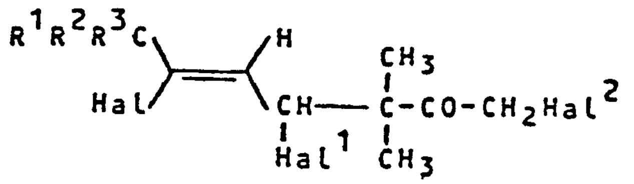

(IX)

in welcher
$R^1$, $R^2$, $R^3$, Hal und $Hal^1$ die oben angegebenen Bedeutungen haben und
$Hal^2$ für Chlor oder Brom steht,
mit Basen der Formel (X),
MO-Z (X)
in welcher
M für ein Aequivalent eines Alkali- oder Erdalkalimetallions steht und
Z für Wasserstoff oder $C_1$-$C_4$-Alkyl steht,
umsetzt und gegebenenfalls aus den so erhältlichen Säuren, Estern oder Salzen in an sich bekannter Weise die Säurehalogenide herstellt.

Schädlingsbekämpfungsmittel, gekennzeichnet durch einen Gehalt an mindestens einem 2,2-Dimethyl-3-(2-halogenvinyl)-cyclopropancarbonsäureester der. Formel (I), gemäß Anspruch 1.

7. Verwendung von 2,2-Dimethyl-3-(2-halogenvinyl)-cyclo-propancarbonsäureester der Formel (I) gemäß Anspruch 1 zur Bekämpfung von Schädlingen.

8. Verfahren zur Herstellung von Schädlingsbekämpfungsmitteln, dadurch gekennzeichnet, daß man 2,2-Dimethyl-3-(2-halogenvinyl)-cyclopropancarbonsäureester der Formel (I) gemäß Anspruch 1 mit Streckmitteln und/oder oberflächenaktiven Mitteln vermischt.

## Claims

1. 2,2-Oimethyl-3-(2-halogeno-vinyl)-cyclopropane-carboxylic acid esters of the formula (I)

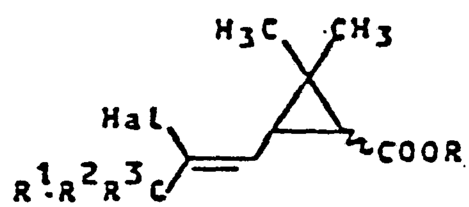

(I)

in which
$R^1$ and $R^2$ are identical or different and represent alkyl or halogenoalkyl,
$R^3$ represents alkyl or the grouping $-(CH_2)_nR^4$,
in which
n represents 0 or 1,
$R^4$ represents halogenoalkyl, phenyl optionally substituted by fluorine, chlorine, bromine, methyl, tert.-butyl, methoxy, methylthio, trifluoromethyl and/or trifluoromethoxy, or the grouping $-XR^5$.
in which
X represents oxygen or sulphur and
$R^5$ represents alkyl, halogenoalkyl, or phenylalkyl or phenyl, optionally substituted by fluorine, chlorine, bromine, methyl, tert.-butyl, methoxy, methylthio, trifluoromethyl and/or trifluoromethoxy, Hal represents fluorina, chlorine or bromine and R represents a radical $-CHR^6R^7$, in which $R^6$ representa hydrogen or CN and $R^7$ represents 3-phenoxyphenyl, 4-fluoro-3-phenoxyphenyl or pentafluorophenyl.

2. Process for the preparation of compounds of the formula (I),
in which
$R^1$ and $R^2$ are identical or different and represent alkyl or halogenoalkyl,
$R^3$ represents alkyl or the roupin $-(CH_2)R^4$
in which
n represents 0 or 1
$R^4$ represents halogenoalkyl, phenyl optionally substituted by fluorine, chlorine, bromine, methyl, tert.-butyl, methoxy, methylthio, trifluoromethoxy and/or trifluoromethyl, or the grouping $-XR^5$,
in which

X represents oxygen or sulphur and

$R^5$ represents alkyl, halogenoalkyl, or phenylalkyl optionally substituted by fluorine, chlorine, bromine, methyl, tert.-butyl, methoxy, methylthio, trifluoromethyl and/or trifluoromethoxy,

Hal represents fluorine, chlorine or bromine and

R represents a radical $-CHR^6R^7$, in which $R^6$ represents hydrogen or CN and $R^7$ represents 3-phenoxyphenyl, 4-fluoro-3-phenoxypheny or pentafluorophenyl,

characterised in that 2,2-dimethyl-3-(2-halogeno-vinyl)-cyclopropanecarboxylic acids of the formula (II)

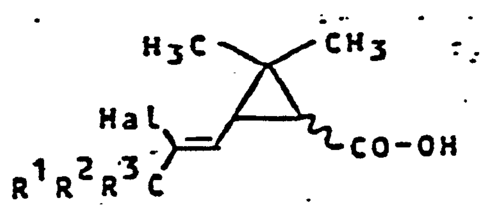

(II)

in which

$R^1$, $R^2$, $R^3$ and Hal have the abovementioned meanings, or reactive derivatives thereof, are reacted with alcohols of the formula (III)

in which

R has the abovementioned meaning, or with reactive derivatives thereof, if appropriate in the presence of acid acceptors, if appropriata in the presence of catalysts and, if appropriate in the presence of diluents.

3. Compounds of the formula (I) according to Claim 1,

in which

$R^1$ and $R^2$ are identical or different and represent methyl, ethyl, chloromethyl, chloroethyl, fluoromethyl or fluoroethyl,

$R^3$ represents methyl, ethyl, n-propyl, i-propyl, n-butyl, n-pentyl n-hexyl or the grouping

in which

n represents 0 or 1,

$R^4$ represents chloromethyl, fluoromethyl, phenyl optionally substituted by fluorine, chlorine, bromine, methyl, methoxy, trifluoromethyl or trifluoromethoxy, and the grouping $XR^5$,

in which

X represents oxygen or sulphur and

$R^5$ represents methyl, ethyl, trifluoromethyl, trichloromethyl, and benzyl or phenyl, optionally substituted by fluorine, chlorine, bromine, methyl, methoxy, trifluoromethyl and/or trifluoromethoxy, and Hal represents chlorine.

4. 2,2-Dimethyl-3-(2-halogenovinyl)-cyclopropane-carboxylic acids and their derivatives of the formula II b

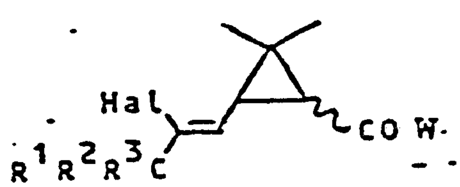

II b

in which

$R^1$, $R^2$, $R^3$ and Hal hava tha meaning given in Claim 1 and

W represents OH, halogen or $C_{1-4}$-alkoxy.

5. Process for the preparation of tha 2,2-dimethyl-3-(2-halogeno-vinyl)-cyclopropane-carboxylic acids and their derivatives of the formula (II b), characterised in that vinyl aldehydes of the formula (VI),

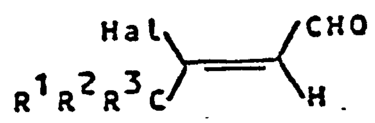

(VI)

in which

$R^1$ $R^2$, $R^3$ and Hal have the meanings given in Claim 1, are reacted with methyl-butan-3-one of the formula (II)

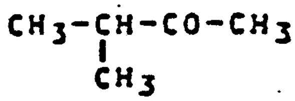

(VII)

in the presence of hydrohalic acids, the 4,4-dimethyl-3-halogeno-1-hexen-5-ones thereby obtainable, of the formula (VIII)

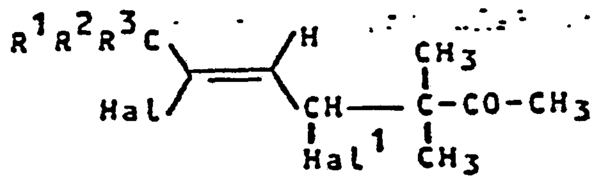

(VIII)

in which
$R^1$, $R^2$, $R^3$ and Hal have the abovementioned meanings and
$Hal^1$ represents chlorine or bromine, are halogenated and the compounds thereby obtainable, of the formula (IX)

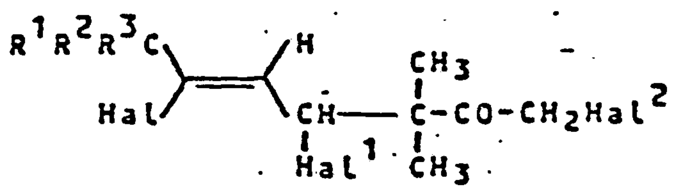

(IX)

in which
$R^1$, $R^2$, $R^3$, Hal and $Hal^1$ have the abovementioned meanings and
$Hal^2$ represents chlorine or bromine, are reacted with bases of the formula (X)
MO-Z (X)
in which
M represents one equivalent of an alkali metal or alkaline earth metal ion and
Z represents hydrogen or $C_1$-$C_4$-alkyl, and, if desired, the acid halides are prepared in a manner known per se from the acida, estera or salts thus obtainable.

6. Agents for combating pests, characterised in that thay contain at-least one 2,2-dimethyl-3-(2-halogeno-vinyl)cyclopropanecarboxylic acid ester of tha formula (I), according to Claim-1.

7. Use of 2,2-dimathyl-3-(2-halogenovinyl)-cyclopropane-carboxylic acid ester of tha formula (I) according to Claim 1 for combating pasts.

8. Process for the preparation of agents for combating peats, characterised in-that 2,2-dimethyl-3-(2-halogenovinyl)cyclopropanecarboxylic acid esters of the formula (I) according to Claim 1 are mixed with extenders and/or surface-active agents.

**Revendications**

1. Esters d'acides diméthyl-2,2 (halogène-2 vinyl)-3 cyclopropanecarboxylique de formule (I)

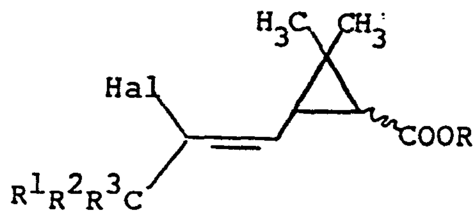

(I)

dans laquelle

$R^1$ et $R^2$ sont identiques ou différents et représentent un alkyle ou un halogénoalkyle,

$R^3$ représente un alkyle ou le groupement $-(CH_2)_nR^4$ où n est 0 ou 1,

$R^4$ représente un halogénoalkyle, un phényle éventuellement substitué par du fluor, du chlore, du brome, un méthyle, un tert-butyle, un méthoxy, un méthylthio, un trifluorométhyle et/ou un trifluorométhoxy ou le groupement $-XR^5$ où

X représente l'oxygène ou le soufre et

$R^5$ représente un alkyle, un halogénoalkyle, un phényle éventuellement substitué par du fluor, du chlore, du bione, un méthyle, un tert-butyle, un méthoxy, un méthylthio, un trifluorométhyle et/ou un trifluorométhoxy ou un phénylakyle,

Hal representa le fluor, le chlore ou le brome et

R représente un reste $-CHR^6R^7$ dans lequel $R^6$ est l'hydrogène ou CN et $R^7$ est un phenoxy-3 phényle, un fluoro-4 phénoxy-3 phényle ou un pentafluorophényle.

2. Procédé pour la préparation de composés de formule (I), dans laquelle

$R^1$ et $R^2$ sont identiques ou différents et représentent un alkyle ou un halogénoalkyle,

$R^3$ représente un alkyle ou le groupement $-(CH_2)$ $R^4$ où n est 0 ou 1,

$R^4$ représente un halogenoalkyle un phényle éventuellement substitué par du fluor, du chlore, du brome, un méthyle, un tert-butyle, un méthoxy, un méthylthio, un trifluorométhyle et/ou un trifluorométhoxy ou le groupement $-XR^5$ où

X représente l'oxygène ou le soufre et

$R^5$ représente un alkyle, un halogénoalkyle, un phényle éventuellement substitué par du fluor, du chlore, du brome, un méthyle, un tert-butyle, un méthoxy, un méthylthio, un trifluorométhyle et/ou un trifluorométhoxy ou un phénylalkyle,

Hal représente le fluor, le chlore ou le brome et

R représente un reste $-CHR^6R^7$ dans lequel $R^6$ est l'hydrogène ou CN et $R^7$ est un phénoxy-3 phényle, un fluoro-4 phénoxy-3 phényle ou un pentafluorophényle, caractérisé en ce que l'on fait réagir des acides diméthyl-2,2 (halogène-2 vinyl)-3 cyclopropanecarboxyliques de formule (II)

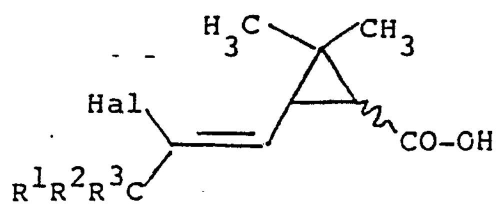

(II)

dans laquelle $R^1$, $R^2$, $R^3$ et Hal ont les significations précédentes ou leurs dérivés réactifs,

avec des alcools de formule (III)

R-OH (III)

dans laquelle R a la signification précédente, ou avec leurs dérivés réactifs, au besoin en présence d'accepteurs d'acides, éventuellement en présence de catalyseurs et éventuellement en présence de diluants.

3. Composés de formule (I) selon la revendication 1 dans laquelle

$R^1$ et $R^2$ sont identiques ou différents et représentent un méthyle, un éthyle, un chlorométhyle, un chloroéthyle, un fluorométhyle ou un fluoroéthyle;

$R^3$ représente un méthyle un éthyle un n-propyle, un i-propyle, un n-butyle, un n-pentyle, un n-hexyle ou le groupement $-(CH_2)_nR^4$ où n est 0 ou 1,

$R^4$ représente un chlorométhyle, un fluorométhyle, un phényle éventuellement substitué par du fluor, du chlore, du brome, un méthyle, un méthoxy, un trifluorométhyle ou un trifluorométhoxy, ainsi que le groupement $XR^5$ où

X est l'oxygène ou le soutre et

$R^5$ représente un méthyle, un éthyle, un trifluorométhyle, un trichlorométhyle ainsi qu'un phényle éventuellement substitué par du fluor, du chlore, du brome, un méthyle, un méthoxy, un trifluorométhyle et/ou un trifluorométhoxy ou un benzyle et

Hal représente le chlore.

4. Acides diméthyl-2,2 (halogène-2 vinyl)-3 cyclopropanecarboxyliques et leurs dérivés de formule IIb

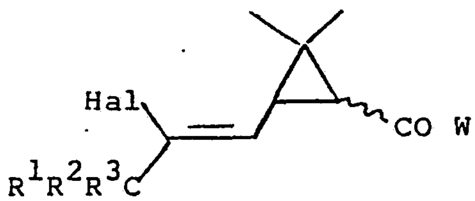

IIb

dans laquelle

$R^1$, $R^2$, $R^3$ et Hal ont la signification indiquée dans la revendication 1 et

W représente OH, un halogène, un alcoxy en $C_1$ à $C_4$.

5. Procédé pour la préparation des acides di-méthyl-2,2 (halogène-2 vinyl)-3 cyclopropanecarboxyliques et de leurs dérivés de formule (IIb), caractérisé en ce que l'on fait réagir des aldéhydes vinyliques de formule (VI)

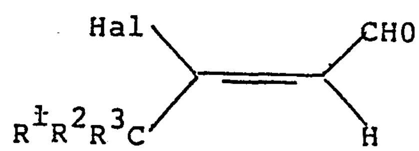

(VI)

dans laquelle $R^1$, $R^2$, $R^3$ et Hal ont les significations indiquées dans la revendication 1, avec de la méthylbutanone-3 de formule (VII)

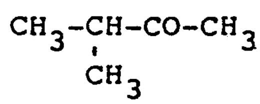

(VII)

en présence d'hydracides, que l'on halogène les diméthyl-4,4 halogène-3 hexène-1-ones-5 ainsi obtenues de formule (VIII)

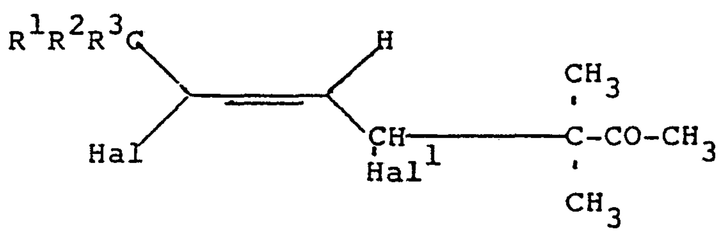

(VIII)

dans laquelle $R^1$, $R^2$, $R^3$ et Hal ont les significations précédentes et

$Hal^1$ est le chlore ou le brome, que l'on fait réagir les composés ainsi obtenus de formule (IX)

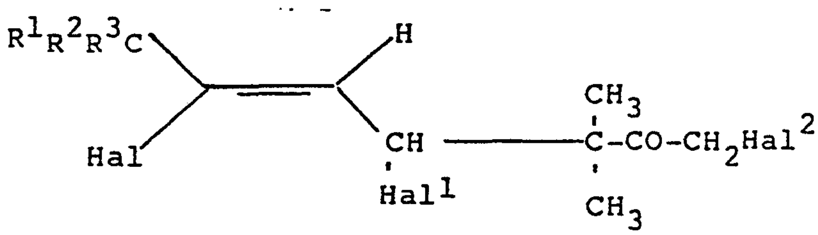

(IX)

dans laquelle $R^1$, $R^2$, $R^3$, Hal et $Hal^1$ ont les significations précédentes et $Hal^2$ est le chlore ou le brome, avec des bases de formule (X)

MO-Z (X)

dans laquelle

M est un équivalent d'ion de métal alcalin ou alcalino-terreux et

Z est l'hydrogène ou un alkyle en $C_1$ à $C_4$ et qu'éventuellement on prépare de façon connue en soi les halogénures d'acides à partir des acides, des esters ou des sels ainsi obtenus.

6. Pesticides, caractérisés par une teneur en au moin; un ester d'acide diméthyl-2,2 (halogène-2 vinyl) -3 cyclopropanecarboxylique de formule (I), selon la revendication 1.

7. Utilisation des esters d'acides diméthyl -2,2 (halogène-2 vinyl)-3 cyclopropanecarboxyliques de formule (I) selon la revendication 1 pour la lutte contre les insectes nuisibles.

8. Procédé de préparation de pesticides, caractérisé en ce qu'on mélange des esters d'acides diméthyl-2,2 (halogène-2 vinyl)-3 cyclopropanecarboxyliques de formule (I) selon la revendication 1 avec des diluants et/ou des agents tensioactifs.